# EUROPEAN PATENT APPLICATION

(11) **EP 3 593 634 A1**
(43) Date of publication of application: **15.01.2020**
(21) Application number: 18182440.0
(22) Date of filing: 09.07.2018
(51) Int. Cl.: A01K 11/00, A61B 5/026, A61B 5/1455, A61B 5/00, A01K 29/00, A61B 5/145, A61B 5/1459

(54) **AN EAR TAG AND ANIMAL MONITORING SYSTEM**

(71) Applicant: Stevens Consultant BVBA, 3945 Ham (BE)
(72) Inventor: Stevens, Hans, 3945 Ham (BE)
(74) Representative: IP HILLS NV

(57) **Abstract**

An ear tag (1) suitable for monitoring an animal comprises an ear plug (10). The ear plug (10) comprises a base (11) and a shaft (12) extending upwards from the base (11). A piercing pin (31) is mounted at the end of the shaft (12) and dimensioned to pierce the ear of the animal such that the shaft (12) becomes surrounded by ear tissue (460). The ear plug (10) further comprises a first light pipe (17; 413) designed to guide light from a light source (421) near a first base opening (13) towards ear tissue (460) of the animal near a first shaft opening (15). The ear plug (10) also comprises a second light pipe (18; 414) designed to guide reflected light from ear tissue (460) of the animal near a second shaft opening (16) to a light detector (422) near a second base opening (14).

## Description

### Field of the Invention

The present invention generally relates to health state monitoring of animals, for instance cattle on a farm. More particularly, the invention relates to wearable devices for monitoring physiological parameters indicative for the health state of an animal, like for instance the haemoglobin value, the blood pressure, the blood oxygen saturation, the heart rate, the body temperature, etc., in a non-invasive manner.

### Background of the Invention

In order to be able to assess the health state or physiological condition of animals, it is important to be able to regularly measure physiological parameters thereof. In certain situations, this may even be mandatory by animal protecting law. As an example, upon birth of a calf, it is decided if the calf will be grown to produce white meat or to produce rose meat. A white meat calf will follow a milk diet as a result of which its haemoglobin value will reduce over time. In the interest of the calfs health status, its haemoglobin value should not drop below a certain predefined threshold, and a farmer growing white meat calves is therefore obliged to regularly monitor the haemoglobin value of each calf. At present, this is often done through blood samples. Taking blood samples however is a manual and invasive method that is cumbersome, time consuming and expensive. Moreover, the blood samples must be collected and physically transported to a laboratory for individual analysis. Thereafter, the results must be reported back. In case of a haemoglobin value below the threshold, the calfs diet must be adjusted for instance to an iron rich diet. Apart from the fact the technique is invasive, the entire chain of physically obtaining, transporting and analysing blood samples to monitor the health state of animals is susceptible for fraud or deceit.

In order to avoid invasive techniques wherein blood, tissue or vessel samples of animals are collected, many wearable devices have been developed to monitor the state of health.

Unites States Patent US 8,251,903 B2 entitled "Noninvasive Physiological Analysis Using Excitation-Sensor Modules and Related Devices and Methods" describes a wearable pulse oximetry sensor that integrates energy emitters like for instance LEDS, energy detectors like for instance photodiodes, a processor and wireless transmitter in an earpiece that is worn by the organism whose physiological condition is assessed. The earpiece, shown in Fig. 11 and Fig. 13 of US 8,251,903 B2 is designed to be worn by a human being, has a rather complex shape and structure, is difficult to manufacture and expensive. In particular in the industry of cattle breeding where a single farm holds hundreds of animals that need to be monitored simultaneously, complex and expensive wearables are not considered. The earpiece known from US 8,251,903 B2 further is removable. Even if its complexity and cost would be reduced, it would leave the door open to fraud or deceit.

United States Patent US 8,600,469 B2 entitled "Medical Sensor and Technique for Using the Same" describes a pulse oximetry sensor with improved accuracy. The inner surface of the sensor known from US 8,600,469 B2 has surface features, for instance protrusions, that reduce the amount of ambient light that may impinge the detection of light. Although optical noise is reduced, the sensor requires light to travel through an undetermined amount of organic material because light emitted by a light emitter (40 in Fig. 2 of US 8,600,469 B2) must travel through a finger, toe or ear to be captured by an optical detector (42 in Fig. 2 of US 8,600,469 B2) facing the light emitter. As a result, the sensor will remain vulnerable to noise. This known sensor is further disadvantageous because the inner surface with protrusions is difficult to manufacture, hence increasing the price of the sensor. The sensor also is removable and therefore provides no adequate protection against fraud or deceit.

The article "Wearable Sensor System for Wireless State-of-Health Determination in Cattle" from the authors L. Nagl et al., published in the IEEE EMBS Conference Proceedings 2003, addresses the problem of cost, size and power consumption of pulse oximeters used to monitor the blood oxygen saturation and pulse rate of a cow. The wearable described in this article consists of a custom-designed ear tag that will permanently attach to the cow's ear and a so called BMOO unit that integrates other functions like data processing, data storage, GPS positioning, Bluetooth transmission, etc. and that fits into a cow-bell, a collar or a belt fixed around the animal's thorax. Whereas Nagl et al. fail to describe how the permanently attached ear tag should be realized, their BMOO unit is voluminous, susceptible to damage, fraud and deceit.

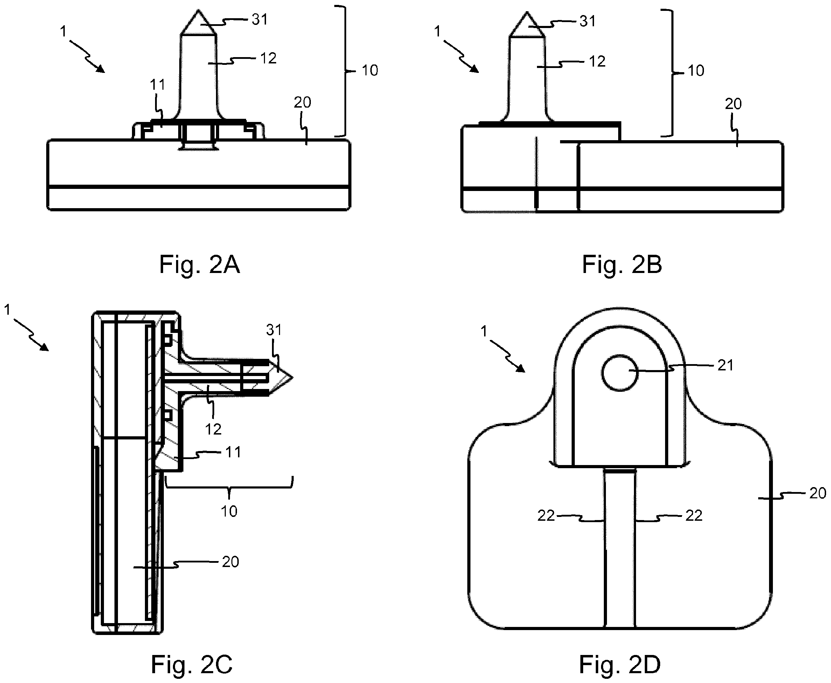

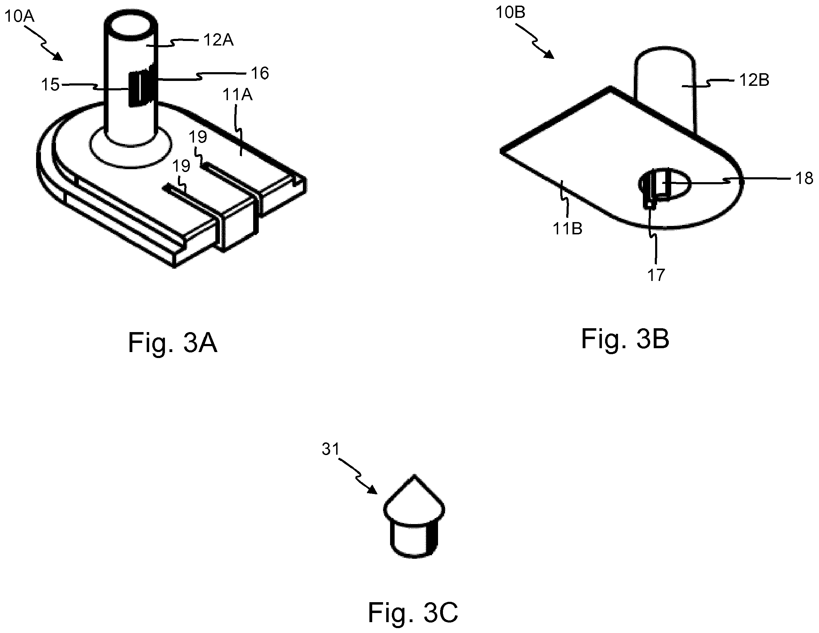

United States Patent US 6,144,867 entitled "Self-Piercing Pulse Oximeter Sensor Assembly" describes another pulse oximeter sensor that can be attached to the human or animal ear through piercing. The light source (20 in Fig. 3) and optical detector (22 in Fig. 3) that form part of this sensor are disposed on a flexible base (26 in Fig. 2), on opposite sides of the earlobe that is pierced. The sensor known from US 6,144,867 is inaccurate as it relies on the detection of light that has travelled through an unspecified amount of earlobe tissue. As a result of the flexible base, the optical detector may not always be well-aligned with the light source, and the measurements are sensitive to movement, further reducing its reliability.

It is an object of the present invention to disclose an improved device for monitoring an animal. More particularly, it is an objective to disclose a device with improved accuracy, reliability and protection against fraud. It is a further objective to provide a device that is small in size, complexity and cost such that it can be mass-produced and used in the cattle breeding industry to monitor large amounts of animals in a non-invasive, animal-friendly manner.

### Summary of the Invention

According to the present invention, one or more of the above-defined objectives are achieved by the ear tag defined by claim 1, suitable for monitoring an animal, the ear tag comprising an ear plug wherein the ear plug comprises:
- a base and a shaft extending upwards from the base, wherein the base comprises a first base opening and a second base opening, and wherein the shaft comprises a first shaft opening and a second shaft opening;
- a piercing pin mounted at the end of the shaft, the piercing pin being dimensioned to pierce an ear of the animal such that the shaft becomes surrounded by ear tissue;
- a first light pipe between the first base opening and the first shaft opening, the first light pipe being designed to guide light from a light source near the first base opening towards ear tissue of the animal near the first shaft opening; and
- a second light pipe between the second base opening and the second shaft opening, the second light pipe being designed to guide reflected light from ear tissue of the animal near the second shaft opening to a light detector near the second base opening.

Thus, the invention concerns an ear tag that at least comprises an ear plug suitable to be pierced through the animal's ear. The ear plug thereto comprises a shaft with piercing pin at one end and a base at the opposite end. Whereas the piercing pin is preferably made of metal, the shaft and base shall typically be made of a plastic and preferably form a single moulded piece. The shaft shall have a substantially cylindrical shape, the curved surface of which becomes surrounded by ear tissue once the ear plug is pierced through the animal's ear. Consequently, the base of the ear plug is the sole part that remains visible in the animal's earcup. In accordance with the present invention, the ear plug has a first light pipe, embedded in the base and shaft. This first light pipe is dimensioned, shaped and oriented to bring light from a first opening in the base surface to a first opening in the shaft's curved surface. This way, light from a light source that is located near the first base opening and that emits this light into the first base opening, will be guided through the first light pipe towards the first shaft opening where this light will enter the ear tissue of the animal. In accordance with the present invention, the ear plug further has a second light pipe, embedded in the base and shaft. This second light pipe is dimensioned, shaped and oriented to bring light from a second opening in the shaft's curved surface to a second opening in the base surface. The second shaft opening is preferably located near the first shaft opening, such that a portion of the light leaving the first shaft opening, entering the animal's ear tissue and being reflected there, enters the second shaft opening. Such reflected light shall than be guided through the second light pipe towards the base of the ear plug where it leaves the base via the second base opening. A light detector located at the second base opening and oriented towards the second base opening, consequently will be able to capture reflected light that can further be analysed in order to derive therefrom various physiological parameters indicative for the animal's health state. The first light pipe and second light pipe are preferably made of a second plastic that is transparent for the wavelengths of the light that will be used to monitor certain physiological parameters of the animal. It is noticed that, depending on the application, this light may be human-visible or human-invisible light, for instance infrared or ultraviolet light. The light may have a single wavelength or, alternatively, light of plural wavelengths may be combined. The light pipes, made of a second plastic, may be interconnected in the base of the ear plug, such that the light pipes can be made as a single moulded piece. A two-component moulding process may be used to manufacture embodiments of the ear plug according to the present invention.

The ear plug is easy and cheap to mass-manufacture, for instance through a two-component moulding process. The ear plug therefore must not be recuperated and therefore can be securely and irremovably attached to the animal's ear, for instance using a clips that fits the pin. The ear plug further enables to bring light from any light source to ear tissue of the animal in a way that is non-sensitive to movement of the animal and non-sensitive to ambient light. Similarly, the ear plug enables to bring reflected light from the animal's ear to any light detector in a way that is non-sensitive to movement of the animal and non-sensitive to ambient light. A wide variety of light sources and corresponding light detectors consequently can be deployed to capture signals that allow to determine various physiological parameters of the animal, like for instance the animal's blood pulse rate, body temperature, heart rate variation, haemoglobin value, etc. The light source(s) and light detector(s) may be integrated in the ear tag or, alternatively, may be integrated in an optical sensor that is removably attached to the ear plug. In the latter situation where the light source and light detector form part of a removable optical sensor, the more expensive components are housed in a device - the optical sensor - that may be recuperated and reused to monitor plural animals, whereas the cheap ear plug remains irremovably attached to the animal. The ear plug may provide an instrument against fraud as it will serve as an identification of the animal where it is securely and irremovably attached to throughout the animal's lifetime.

In embodiments of the ear tag according to the invention, as defined by claim 2, the ear plug further comprises a unique machine-readable plug identifier.

Thus, in advantageous embodiments, the ear plug has a unique machine-readable, plug identifier, like for instance a Radio-Frequency Identifier or RFID, integrated. Such identifier can serve as unique identification of the animal, and/or can be used to sign any signals that are captured from the animal. This way, the ear plug prevents against several types of fraud or deceit: signals or physiological parameters calculated therefrom can no longer be manipulated when they are signed by the unique plug identifier, and signals or physiological parameters can no longer be associated with other animals because the unique plug identifier that is used to sign the signals and physiological parameters is linked to a single animal throughout the lifetime of that animal. The unique plug identifier is machine-readable by a machine brought in the vicinity of the animal, for instance and RFID reader or Near Field Communications (NFC) reader. Alternatively or additionally, the unique plug identifier may be readable through a machine that is physically connected to the ear plug, like for instance the above mentioned optical sensor if this optical sensor is equipped with an interface that enables exchange of the unique plug identifier.

In embodiments of the ear tag according to the invention, as defined by claim 3, the base and shaft form a single moulded piece of a first, non-transparent material.

Indeed, as mentioned above, the base and shaft of the ear plug are preferably made of a single material, for instance a plastic. This material is non-transparent to light in the wavelength range that will be used to monitor the animal, such that leakage of light travelling through the first light pipe and leakage of reflected light travelling through the second light pipe is minimized. This way, the accuracy and reliability of the ear tag for animal monitoring is further enhanced. When produced as a single moulded piece, the base and shaft can be given a smooth, seamless external surface that cannot injure the animal, collect dirt or hair. Moreover, a moulding process is very suitable for cheap mass-production that addresses the needs of the cattle breeding industry.

In embodiments of the ear tag according to the invention, as defined by claim 4, the first light pipe and the second light pipe form part of a second moulded piece of a second, light transparent material.

Indeed, as mentioned above, the first and second light pipes may be made of a second, plastic material that is optimally transparent for the wavelengths that will be used to monitor the animal. This way, the accuracy and reliability of animal monitoring is further improved, and the risk for contamination through dirt or hair that may negatively impact the monitoring is also minimized. The light pipes may constitute a second moulded piece that fits into the first moulded piece representing the base and shaft of the ear plug. Cheap mass-production of a disposable ear plug that is used to monitor a single animal throughout its life time, is made possible this way. The first and second moulded pieces may be manufactured through separate mould processes or they may be manufactured through a two-component injection moulding process wherein the first moulded piece obtained after injection of the first, non-transparent plastic material serves as mould for the second, transparent material, such that a single product made of two (or more) plastics is made.

In embodiments of the ear tag according to the invention, as defined by claim 5, the ear plug comprises a first air gap surrounding the first light pipe and/or a second air gap surrounding the second light pipe.

Such embodiments of the ear plug wherein the light pipes are separated from the base and shaft through an airgap, are advantageous because the air gap improves the refractive index of light and consequently the efficiency of the ear tag when used to monitor physiological parameters of the animal through optical measurements.

In embodiments of the ear tag according to the invention, as defined by claim 6, the first light pipe comprises:
- a first pipe portion extending from the first base opening substantially parallel to the longitudinal axis of the shaft;
- a second pipe portion extending from the first shaft opening substantially parallel to a radial axis of the shaft; and
- a first slanted, light-reflective wall, in between the first pipe portion and the second pipe portion,
and the second light pipe comprises:
- a third pipe portion extending from the second base opening substantially parallel to the longitudinal axis of the shaft;
- a fourth pipe portion extending from the second shaft opening substantially parallel to a radial axis of the shaft; and
- a second slanted, light-reflective wall, in between the third pipe portion and the fourth pipe portion.

Indeed, to connect the first base opening in the base surface with the first shaft opening in the curved surface of the shaft, the first light pipe either may be bended or, more preferably, may be made of two pipe portions that are oriented substantially perpendicular to each other. Where the two pipe portions meet each other, a slanted wall may be foreseen, for instance oriented under an angle of 45 degrees vis-à-vis the axes of the two pipe portions, in order to optimally couple light received from a light source facing the first base opening from one pipe portion into the other pipe portion. In a similar way, to connect the second base opening in the base surface with the second shaft opening in the curved surface of the shaft, the second light pipe either may be bended or, more preferably, may be made of two pipe portions that are oriented substantially perpendicular to each other and interconnected through a slanted wall under an angle of 45 degrees. This way, also reflected light is optimally guided from one pipe portion into the other pipe portion and therefore maximally coupled back from the ear tissue to an optical detector facing the second base opening.

In embodiments of the ear tag according to the invention, as defined by claim 7, the shaft has a length shorter than twice the thickness of the ear.

Indeed, to ensure that light leaving the first shaft opening is coupled into ear tissue to be reflected therein while considering that ears of animals have varying thicknesses, the length of the shaft must be limited to at most two times the average thickness of the animal's ear. This way, the animal's ear surrounds the shaft along at least half of its length, thereby minimizing the risk that the first and/or second shaft opening are left uncovered by ear tissue as a result of which monitoring the animal would be hampered or sub-optimal.

Embodiments of the ear tag according to the invention, as defined by claim 8, further comprise a sensor wherein the sensor comprises:
- the light source, positioned and dimensioned to emit light into the first base opening;
- the light detector, positioned and dimensioned to receive light from the second base opening; and
- a battery configured to power the light source and the light detector.

Thus, a light source, light detector and battery powering the light source and light detector may jointly form an optical sensor that is connected releasably or non-releasably to the ear plug and makes use of the light pipes in the ear plug to reliably perform optical measurements that allow to determine various physiological parameters of the animal.

In embodiments of the ear tag according to the invention, as defined by claim 9, the sensor is removably attachable to the base of the ear plug through a click mechanism.

The optical sensor may for instance have a housing with mechanical click mechanism that allows to releasably fasten the optical sensor to the ear plug in such a manner that the light source faces the first base opening of the ear plug and the light detector faces the second base opening of the ear plug. This way, the optical sensor that contains the more expensive components becomes dummy-proof attachable to an ear plug pierced in an animal's ear, and becomes removable for recuperation and use on another animal, for battery replacement, maintenance, read-out or any other purpose.

In embodiments of the ear tag according to the present invention, defined by claim 10, the sensor further comprises a unique machine-readable sensor identifier.

Hence, also the optical sensor may carry a unique machine-readable identifier, named the sensor identifier. The sensor identifier may for instance also be an RFID. This unique sensor identifier may be combined with the unique plug identifier to sign the signals or physiological data derived therefrom, such that all data obtained through monitoring can be related to a particular sensor and to a particular animal. If a sensor is used on multiple animals, this will be logged through its sensor identifier being associated sequentially with different plug identifiers, thereby further reducing the risk for fraud or deceit.

In embodiments of the ear tag according to the invention, as defined by claim 11, the sensor further comprises a position determination unit.

Indeed, in advanced embodiments of the ear tag, the sensor has position determination technology aboard, like for instance a GPS (Global Positioning System) receiver. The presence of position determination technology enables to trace the location, for instance the GPS coordinates, of a sensor, further reducing the risk for fraud of deceit. At any time, the location of a sensor may be inspected. Position determination technology also allows to log signals or physiological data derived therefrom together with the location where such signals were measured.

Embodiments of the ear tag according to the present invention, as defined by claim 12, further comprise an accelerometer.

Hence, movements of the animal may be monitored and logged in conjunction with signals captured by the sensor and/or physiological parameter values derived therefrom.

In embodiments of the ear tag according to the invention, as defined by claim 13, the sensor further comprises at least one wireless transceiver and a wireless transceiver controller coupled to one or several of the light source and/or the light detector and/or the unique machine-readable identifier and/or the position determination unit and/or the accelerometer.

Indeed, the different sensor components involved in the collection of data like the light source, light detector, its identifier, location unit and/or accelerometer, are preferably coupled to a wireless transceiver controller integrated in the sensor to control transmission and reception of data in a wireless manner. Preferably, the sensor has a short range wireless transceiver, for instance a Bluetooth transceiver enabling data exchange between the sensor and a Bluetooth capable device in close vicinity of the animal, and a long range wireless transceiver, for instance an 868 MHz radio transceiver allowing exchange of data between the sensor and data collecting nodes that may for example be located in a farm, 10 kilometres away from the animal.

In embodiments of the ear tag according to the invention, as defined by claim 14, the sensor further comprises a solar cell panel and battery charger configured to charge the battery using solar energy.

To avoid frequent interruptions in the monitoring of an animal as a result of empty batteries and frequent interventions to replace the batteries, the sensor preferably has a small solar cell panel integrated that is used to automatically charge the batteries of the sensor using solar energy that is expected to be available when the animal carrying the ear tag is for instance grazing in a meadow.

In embodiments of the ear tag according to the invention, as defined by claim 15, the sensor further comprises a human visible status light-emitting diode, abbreviated a status LED.

Indeed, the sensor may further be provided with a status LED that indicates a status or condition of the animal. The status LED may be configured and controlled to automatically light up of blink when certain measurements exceed thresholds, when an animal has received a certain treatment, like a preventive vaccination, etc.

Embodiments of the ear tag according the present invention, as defined by claim 16, further comprise a non-removable clip which, when mounted on the piercing pin, secures the ear tag on the ear of said animal.

Indeed, to prevent fraud or deceit, measures must be taken to avoid that the ear plug pierced in the ear of an animal can be removed and pierced into the ear of another animal. This may be realized in embodiments of the invention through a mechanical solution, for instance a clip that is clicked onto the piercing pin and that can only be removed by damaging the clip and/or pin such that the ear plug becomes non-usable on a different animal.

In addition to an ear tag as defined by claim 1, the present invention also relates to an animal monitoring system as defined by claim 17, comprising:
- one or more ear tag, each ear tag being attached to a respective animal;
- one or more data collector comprising a wireless transceiver configured to receive data from the one or more ear tag and a data store configured to temporarily store the data received from the one or more ear tag; and
- one or more blockchain node, coupled to the one or more data collector and configured to receive the data from the one or more data collector and write the data into a data block of a blockchain.

The ear tags according to the invention in other words preferably are used in a monitoring system that further comprises data collectors and blockchain nodes. The ear tags transfer the sampled optical signals regularly to the data collectors. The data collector is a node with a long range wireless transceiver, for instance an 868 MHz radio transceiver, that is configured to receive data from various ear tags attached to animals within the range covered by the wireless transceiver. One or more of these data collectors may be installed per farm, depending on the size of the farm, the area of meadows to be covered, etc. The signal samples may be transferred from the ear tags worn by animals of a farm to the data collector(s) each time measurements take place, or alternatively on request of the data collector(s), or in yet another alternative at a regular pace, for instance each time a predetermined time interval has lapsed. Each data collector further has a storage facility to temporarily store the received signal samples, and a wireless or wired interface towards a blockchain node or gateway. The data collector thus transfers the signal samples to a blockchain gateway whose task it is to add the signal samples to a block of a blockchain. Once the data are written on the blockchain, the data can't be changed anymore. The blockchain consequently provides a further measure against fraud or deceit. Thanks to the blockchain, no trusted party must be involved to verify or approve the data. The blockchain technology inherently provides resistance to fraudulent modifications of the data. Combined with the unique ear plug identifier that serves as an identification of the animal throughout its lifetime, the unique sensor identifier that is linked with the ear plug identifier for the time the sensor is used to monitor an animal, the blockchain ledger is closing the loop for secure, reliable and fraud-resistant monitoring of the health state of animals on large scale. The blockchain is decentralized and every party involved can get an exact copy of the blockchain data.

Embodiments of the animal monitoring system according to the present invention, defined by claim 18, further comprise:
- machine learning technology trained to process the data before or after being written into the blockchain.

Thus, the signals measured by the ear tag according to the invention and stored into a blockchain may be processed, for instance to derive therefrom physiological parameters of the animal. In embodiments of the invention, the processing is done by neural networks trained to derive one or more physiological parameter from the measured signals.

In embodiments of the animal monitoring system according to the invention, as defined by claim 19, the data comprise a photoplethysmogram and the machine learning technology is trained to determine a haemoglobin value for blood of an animal based on the photoplethysmogram.

In one application where the ear tag according to the invention is useful, the reflected light signals captured by the light sensor are used to determine a PPG or photoplethysmogram. This PPG can then serve as input to a neural network trained to derive from the PPH a haemoglobin value for the animal's blood. When the haemoglobin value of the animal's blood has dropped below a certain threshold, for instance because the animal is following a milk diet, the animal's diet may have to be adapted with irony supplements.

### Brief Description of the Drawings

Fig. 1A and Fig. 1B respectively represent a front view and a cross-section view of an embodiment of the ear plug 10 that forms part of the ear tag according to the present invention; Fig. 1C and Fig. 1D respectively represent a front view and bottom view of a first moulded piece 10A that forms part of the ear plug 10 of Fig. 1A-1 B; Fig. 1E represents a cross-section view of a second moulded piece 10B that forms part of the ear plug 10 of Fig. 1A-1B; and Fig. 1F represents a front view of the piercing pin 31 that forms part of the ear plug 10 of Fig. 1A-1 B;
Fig. 2A, Fig. 2B and Fig. 2C respectively represent a front view, a side view, and a cross-section view of an embodiment of the ear tag 1 according to the present invention, comprising the ear plug 10 of Fig. 1A-1F, and a sensor 20; Fig. 2D shows a top view of the sensor 20;
Fig. 3A, Fig. 3B and Fig. 3C respectively represent three-dimensional views of a first moulded piece 10A, a second moulded piece 10B, and a piercing pin 31 jointly constituting an embodiment of the ear plug 10 that forms part of an embodiment of the ear tag 1 according to the invention;
Fig. 4 is a functional block scheme of an embodiment of the ear tag according to the present invention, comprising the ear plug 10 of Fig. 1A-1F and the sensor 20 of Fig. 2A-2D;
Fig. 5 illustrates an embodiment of the animal monitoring system 500 according to the present invention; and
Fig. 6 illustrates use of the present invention in an application where the haemoglobin value of the animal is monitored.

### Detailed Description of Embodiment(s)

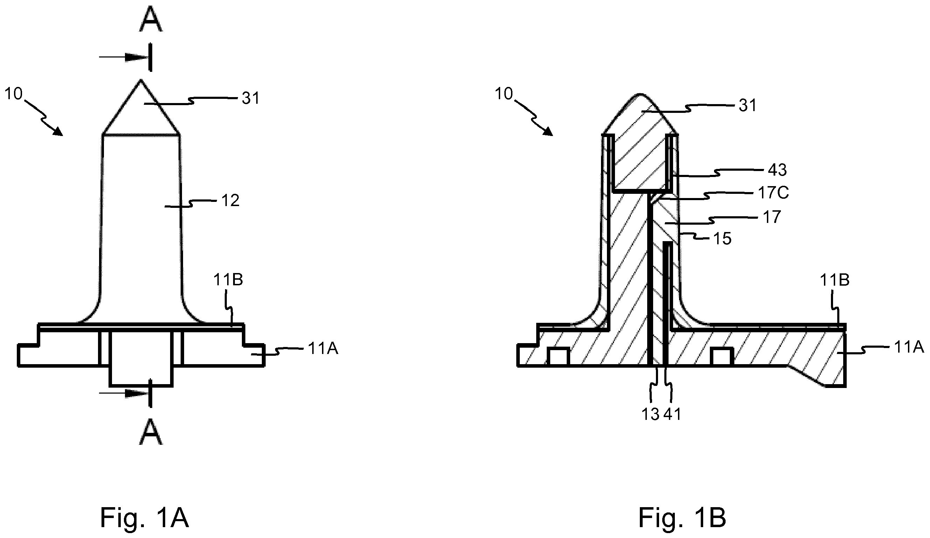

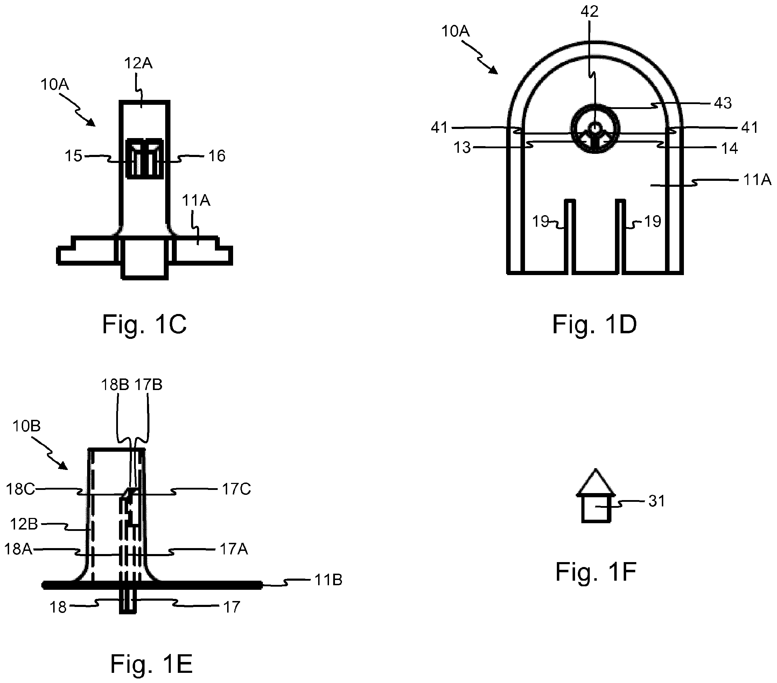

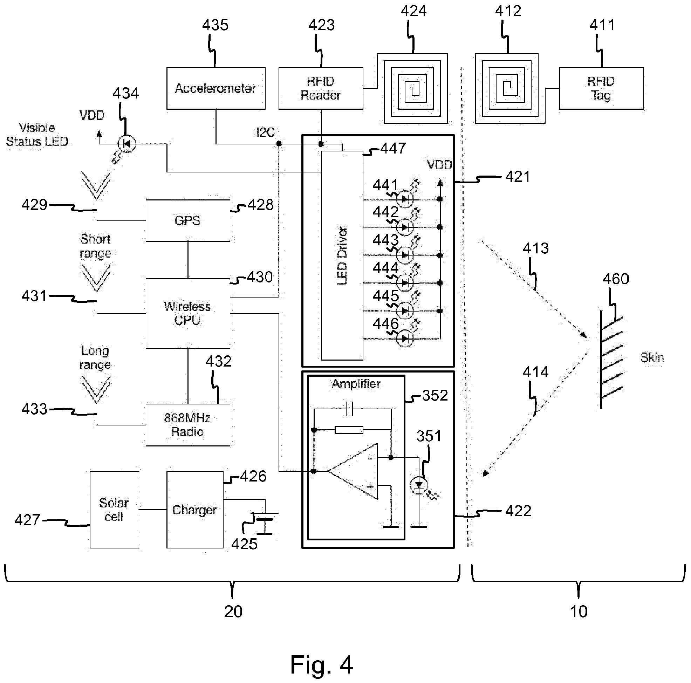

Fig. 1A-1F, Fig. 2A-2D and Fig. 3A-3C jointly illustrate an embodiment of the ear tag 1 according to the present invention. The ear tag 1 comprises an ear plug 10 and a sensor 20. The ear plug 10 consists of three parts: a first moulded piece 10A made of a first non-transparent plastic, a second moulded piece 10B made of a transparent plastic, and a piercing pin 31 made of metal. The second moulded piece 10B fits into the first moulded piece 10A or vice-versa, and the first moulded piece 10A has a sink 43 at its top to hold the piercing pin 31, as a result of which the three parts jointly form ear plug 10 having a base 11, shaft 12 and piercing pin 31. The base 11 consists of a first base part 11A that forms part of the first moulded piece 10A and a second base part 11B that forms part of the second moulded piece 10B. The first base part 11A is non-transparent to the wavelengths that are used by sensor 20. The first base part 11A has a first base opening 13 and a second base opening 14. The second base part 11B is transparent to the wavelengths that are used by sensor 20. The base 11 further provides mechanical connectivity to the sensor 20, for instance through a mechanical slide-and-click mechanism 19. The shaft 12 consists of a first shaft part 12A that forms part of the first moulded piece 10A and a second shaft part 12B that forms part of the second moulded piece 10B. The first shaft part 12A is non-transparent to the wavelengths that are used by sensor 20 and has a curved, substantially cylindrical outer surface. This curved, cylindrical outer surface has a first shaft opening 15 and a second shaft opening 16. The first, non-transparent moulded piece 10A further has a first internal channel that interconnects the first base opening 13 with the first shaft opening 15, and a second internal channel that interconnects the second base opening 14 with the second shaft opening 16. These first and second channels in the first, non-transparent moulded piece 10A are filled with transparent material of the second moulded piece 10B that form a first light pipe 17 and a second light pipe 18 each connected to the second shaft part 12B. This second shaft part 12B provides the curved, cylindrical outer surface of the shaft 12. The first light pipe 17 is designed and dimensioned to interconnect the first base opening 13 and first shaft opening 15 with material that is transparent to the wavelengths used by sensor 20. In the example embodiment illustrated by Fig. 1A-1F, Fig. 2A-2D, Fig. 3A-3C, the first light pipe 17 consists of a first pipe portion 17A, parallel to the longitudinal axis of the shaft 12, a second pipe portion 17B, parallel to a radial axis of the shaft 12, and a slanted wall 17C interconnecting the first pipe portion 17A and the second pipe portion 17B. The slanted wall 17C is oriented under an angle of 45° to maximally couple light from the first pipe portion 17A into the second pipe portion 17B. Similarly, the second light pipe 18 is designed and dimensioned to interconnect the second base opening 14 and second shaft opening 16 with material that is transparent to the wavelengths used by sensor 20. In the example embodiment illustrated by Fig. 1A-1F, Fig. 2A-2D, Fig. 3A-3C, the second light pipe 18 consists of a third pipe portion 18A, parallel to the longitudinal axis of the shaft 12, a fourth pipe portion 18B, parallel to a radial axis of the shaft 12, and a slanted wall 18C interconnecting the third pipe portion 18A and the fourth pipe portion 18B. The slanted wall 18C is oriented under an angle of 45° to maximally couple reflected light from the fourth pipe portion 18B into the third pipe portion 18A. The first moulded piece 10A and second moulded piece 10B may be manufactured through two-component moulding to fit exactly. An air gap 41 may be provided, surrounding the first light pipe 17 or partially surrounding the first light pipe (for instance only the first pipe portion 17A), in order to improve the refractive index of this light pipe 17. Similarly, an air gap 41 may surround or partially surround the second light pipe 18 in order to improve the refractive index thereof. Fig. 1D further shows a central opening 42 in the first moulded piece 10A that is foreseen to enable entrance of a tool that is used to pierce the metal pin 31 through the animal's ear. Fig. 2D at last shows that the housing of optical sensor 20 is provided with an opening 21 and mechanical slide-and-click means 22. The mechanical slide-and-click means 22 of the sensor 20 engage with the mechanical slide and click means 19 of the ear plug 10 such that, once fast-clicked to each other, opening 21 in the sensor housing is facing the first base opening 13 and second base opening 14 of the ear plug 10. The functional components that form part of an example embodiment of sensor 20 will be described in more detail below with reference to Fig. 4.

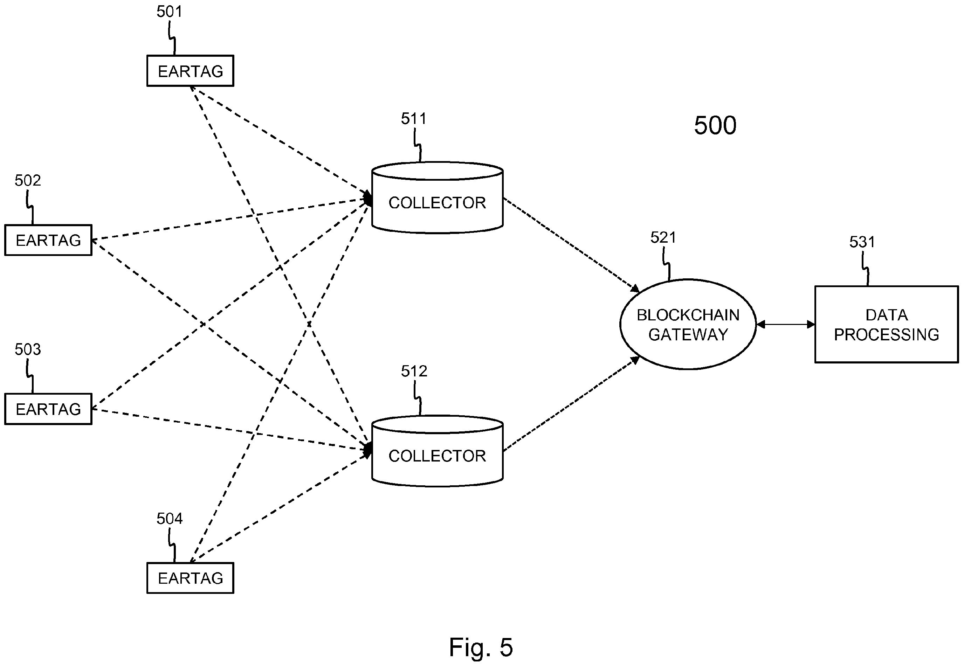

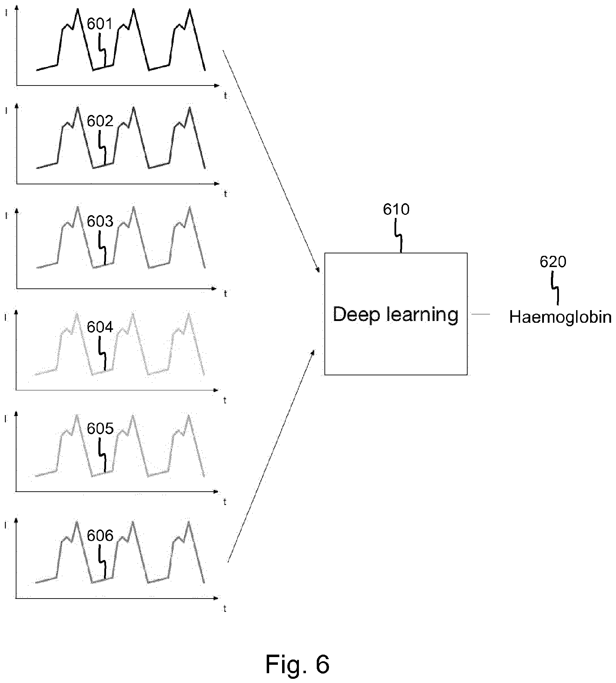

One application wherein the ear tag according to the invention is useful is the monitoring of haemoglobin values for calves that follow a milk diet to become white meat calves. As is illustrated by Fig. 5, such calves may be tagged with ear tags 501, 502, 503, 504 according to the invention. The ear tags, as will be explained below, are equipped with technology that enables photoplethysmography, abbreviated PPG, measurements at multiple wavelengths. As is illustrated by Fig. 6, photoplethysmograms 601, 602, 603, 604, 606, 606 may be measured at six different wavelengths. Returning to Fig. 5, each one of the ear tags 501-504 transmits its six measured PPG signals 601-606 to one or several collector nodes 511, 512 within each reach. The collector nodes 511, 512 receive and temporarily store the PPG signals from all ear tags within their respective reach and forward the received PPG signals to a blockchain gateway 521 that adds these signals to a data block of a blockchain such that the signals are protected against fraud. As is further indicated by Fig. 5, the haemoglobin monitoring system 500 further comprises a data processor 531 that has access to the PPG signals stored in the blockchain and is able to process these PPG according to state-of-the-art algorithms in order to derive therefrom a haemoglobin value 620 for each monitored calf. The data processing 531 may for instance take place in the cloud and rely on a neural network 610 that gathers artificial intelligence through deep learning technologies. The haemoglobin monitoring system 500 illustrated by Fig. 5 and Fig. 6 has as main advantage that it provides a non-invasive, fraud-resistant way to permanently monitor the health state of calves such that the diet of a calf can be adjusted promptly as soon as the measured haemoglobin value of that calf drops below a prescribed minimum threshold.

In order to be able to generate the PPG signals 601-606, the ear tags 501-504 each comprise an ear plug 10 and sensor 20 with functionality as depicted in Fig. 4. The ear plug 10 that is irremovably attached to the calfs ear 460 has an integrated RFID tag 411 with antenna 412. The RFID tag 411 is configured with a unique plug identifier that serves as a unique identification of the calf where it is attached to. This unique plug identifier can be exchanged with sensor 20 via the antenna 412 and/or it can be read by any RFID reader that comes within the vicinity of the calf. The ear plug 10 further has an integrated first light pipe 413 designed to convey light from an opening in its base to an opening in its shaft that is surrounded by ear tissue 460 of the calf. The ear plug 10 also has an integrated second light pipe 414 designed to convey light reflected by ear tissue 460 of the calf from a second shaft opening that is surrounded by the calfs ear tissue 460 to a second base opening. In order to monitor the calfs haemoglobin value, sensor 20 is mechanically clicked onto ear plug 10. Sensor 20 has a light source 421 comprising six LEDs 441, 442, 443, 444, 445 and 446 operated under control of LED driver 447 to emit light having six different wavelengths into light pipe 413. Sensor 20 further has a light detector 422 with a photodiode 351 that captures reflected light at the six different wavelengths from the light pipe 414. The detected signals are amplified by amplifier 352 that forms part of light detector 422. In addition to the light source 421 and the light detector 422, sensor 20 comprises an RFID tag 423 with antenna 424. This RFID tag 423 is configured with a unique sensor ID. This unique sensor ID can be associated with the unique plug ID of RFID tag 411 for the time the sensor 20 is connected to ear plug 10. The senor 20 however can be disconnected from the ear plug 10 and be used to monitor another calf in which case its unique sensor ID will be associated with the unique plug ID of ear plug attached to this new calf that is monitored. This way, sensor 20 can sequentially be used to monitor several calves, and the association between its sensor ID and the respective plug IDs allows to track which calf was monitored by the sensor 20 at any point in time. The signals collected by detector 422 as well as the sensor ID and plug ID will be transmitted regularly, i.e. at regular time intervals or upon request, to collector nodes 511 and/or 512. For the communication with these collector nodes 511, 512, the sensor 20 is equipped with a long range wireless transceiver 432, for instance an 868 MHz radio transceiver 432 with corresponding antenna 433. The long range communication interface may enable to transmit data over a distance of several kilometres to a collector node 511, 512 that may be located at a farm to collect the monitored signals for all animals in the farm. In addition to the long range wireless interface, sensor 20 also has a short range wireless interface 431, for instance a Bluetooth interface, and a wireless central processing unit or wireless CPU 430 that controls wireless transmission over the long range interface 432, 433 and short range interface 431. The short range Bluetooth interface 431 for instance enables any person in the vicinity of the calf with a Bluetooth capable device to read-out the measured signals. Sensor 20 further comprises a GPS unit 428 with satellite receiver 429 able to determine the sensor's location on the basis of GPS satellite signals. The so determined location may be linked to the measurements and communicated together with the measurements via the long range transceiver 432, 433 to a collector node 511, 512 or via the Bluetooth interface 431 to any Bluetooth receiver in the vicinity of the calf. Similarly, the sensor 20 comprises an accelerometer 435 that tracks the calfs movements. Information on the calfs movements during the measurements may also be associated with the measurements and communicated together with the measurements over the long range and/or short range interfaces. The various components of sensor 20 are powered by battery 425. In the embodiment illustrated by Fig. 4, the battery 425 is rechargeable through a charger 426 that obtains electrical energy from a solar cell panel 427 integrated in the sensor 20. A visible status LED 434 under control of LED driver 447 at last may indicate when measured values reach critical values. Alternatively, this visible status LED 434 may be switched on to indicate that the calf has received a certain treatment, for instance a vaccination. This way, the sensors 20 attached to the ear plugs 10 of the different calves can be used to visibly indicate which animals have already received a certain treatment and which have not.

Although the present invention has been illustrated by reference to specific embodiments, it will be apparent to those skilled in the art that the invention is not limited to the details of the foregoing illustrative embodiments, and that the present invention may be embodied with various changes and modifications without departing from the scope thereof. The present embodiments are therefore to be considered in all respects as illustrative and not restrictive, the scope of the invention being indicated by the appended claims rather than by the foregoing description, and all changes which come within the meaning and range of equivalency of the claims are therefore intended to be embraced therein. In other words, it is contemplated to cover any and all modifications, variations or equivalents that fall within the scope of the basic underlying principles and whose essential attributes are claimed in this patent application. As an example, alternative embodiments of the ear plug may have more than two light pipes. A third light pipe may for instance be foreseen to guide infrared light that is used to monitor the animal's body temperature in addition to its haemoglobin value. Other physiological parameters may be monitored based on optical technologies that use one or several wavelengths. Various types of animals may be monitored. The GPS unit may be left out or may be replaced with other positioning technologies. The accelerometer may be left out. A single wireless transceiver may replace the short range and long range wireless transceivers of the above described embodiment and the skilled person will appreciate that the solar cell panel and charger may be left out of embodiments of the sensor that are disposable or wherein the battery is replaceable. In further embodiments, the sensor 20 and ear plug 10 may be integrated in a single housing such that the sensor 20 cannot be disconnected from the ear plug 10. It will furthermore be understood by the reader of this patent application that the words "comprising" or "comprise" do not exclude other elements or steps, that the words "a" or "an" do not exclude a plurality, and that a single element, such as a computer system, a processor, or another integrated unit may fulfil the functions of several means recited in the claims. Any reference signs in the claims shall not be construed as limiting the respective claims concerned. The terms "first", "second", third", "a", "b", "c", and the like, when used in the description or in the claims are introduced to distinguish between similar elements or steps and are not necessarily describing a sequential or chronological order. Similarly, the terms "top", "bottom", "over", "under", and the like are introduced for descriptive purposes and not necessarily to denote relative positions. It is to be understood that the terms so used are interchangeable under appropriate circumstances and embodiments of the invention are capable of operating according to the present invention in other sequences, or in orientations different from the one(s) described or illustrated above.

## Claims

1. An ear tag (1) suitable for monitoring an animal, said ear tag (1) comprising an ear plug (10) wherein said ear plug (10) comprises:
- a base (11) and a shaft (12) extending upwards from said base (11), wherein said base (11) comprises a first base opening (13) and a second base opening (14), and wherein said shaft comprises a first shaft opening (15) and a second shaft opening (16);
- a piercing pin (31) mounted at the end of said shaft (12), said piercing pin (31) being dimensioned to pierce an ear of said animal such that said shaft (12) becomes surrounded by ear tissue (360);
- a first light pipe (17; 413) between said first base opening (13) and said first shaft opening (15), said first light pipe (17; 413) being designed to guide light from a light source (421) near said first base opening (13) towards ear tissue (460) of said animal near said first shaft opening (15); and
- a second light pipe (18; 414) between said second base opening (14) and said second shaft opening (16), said second light pipe (18; 414) being designed to guide reflected light from ear tissue (460) of said animal near said second shaft opening (16) to a light detector (422) near said second base opening (14).

2. An ear tag (1) according to claim 1, wherein said ear plug (10) further comprises a unique machine-readable plug identifier (411).

3. An ear tag (1) according to one of the preceding claims, wherein said base (11) and said shaft (12) form a single moulded piece of a first, non-transparent material.

4. An ear tag (1) according to one of the preceding claims, wherein said first light pipe (17; 413) and said second light pipe (414; 414) form part of a second moulded piece of a second, light transparent material.

5. An ear tag (1) according to one of the preceding claims, wherein said ear plug (10) comprises a first air gap (41) surrounding said first light pipe (17; 413) and/or a second air gap surrounding said second light pipe (18; 414).

6. An ear tag (1) according to one of the preceding claims,
wherein said first light pipe (17; 413) comprises:
- a first pipe portion (17A) extending from said first base opening (13) substantially parallel to the longitudinal axis of said shaft (12);
- a second pipe portion (17B) extending from said first shaft opening (15) substantially parallel to a radial axis of said shaft (12); and
- a first slanted, light-reflective wall (17C), in between said first pipe portion (17A) and said second pipe portion (17B),
and wherein said second light pipe (18; 414) comprises:
- a third pipe portion (18A) extending from said second base opening (14) substantially parallel to the longitudinal axis of said shaft (12);
- a fourth pipe portion (18B) extending from said second shaft opening (16) substantially parallel to a radial axis of said shaft (12); and
- a second slanted, light-reflective wall (18C), in between said third pipe portion (18A) and said fourth pipe portion (18B).

7. An ear tag (1) according to one of the preceding claims, wherein said shaft (12) has a length shorter than twice the thickness of said ear.

8. An ear tag (1) according to one of the preceding claims, further comprising a sensor (20) wherein said sensor (20) comprises:
- said light source (421), positioned and dimensioned to emit light into said first base opening (13);
- said light detector (422), positioned and dimensioned to receive light from said second base opening (14); and
- a battery (425) configured to power said light source (421) and said light detector (422).

9. An ear tag (1) according to claim 8, wherein said sensor (20) is removably attachable to said base (11) of said ear plug (10) through a click mechanism (19).

10. An ear tag (1) according to one of claims 8-9, wherein said sensor (20) further comprises a unique machine-readable sensor identifier (423).

11. An ear tag (1) according to one of claims 8-10, wherein said sensor (20) further comprises a position determination unit (428).

12. An ear tag (1) according to one of claims 8-11, wherein said sensor (20) further comprises an accelerometer (435).

13. An ear tag (1) according to one of claims 8-12, wherein said sensor (20) further comprises at least one wireless transceiver (431; 432, 433) and a wireless transceiver controller (430) coupled to one or several of said light source (421) and/or said light detector (422) and/or said unique machine-readable sensor identifier (423) and/or said position determination unit (428) and/or said accelerometer (435).

14. An ear tag (1) according to one of claims 8-13, wherein said sensor (20) further comprises a solar cell panel (427) and battery charger (426) configured to charge said battery (425) using solar energy.

15. An ear tag (1) according to one of claims 8-14, wherein said sensor (20) further comprises a human visible status light-emitting diode (434), abbreviated a status LED.

16. An ear tag (1) according to one of the preceding claims, further comprising a non-removable clip which, when mounted on said piercing pin (31), secures said ear tag (1) on said ear of said animal.

17. An animal monitoring system (500), comprising:
- one or more ear tag (501, 502, 503, 504) as defined by one of the preceding claims, each ear tag (501, 502, 503, 504) being attached to a respective animal;
- one or more data collector (511, 512) comprising a wireless transceiver configured to receive data from said one or more ear tag (501, 502, 503, 504) and a data store configured to temporarily store said data received from said one or more ear tag (501, 502, 503, 504); and
- one or more blockchain node (521), coupled to said one or more data collector (511, 512) and configured to receive said data from said one or more data collector (511, 512) and write said data into a data block of a blockchain.

18. An animal monitoring system (500) according to claim 17, further comprising:
- machine learning technology (610) trained to process said data before or after being written into said blockchain.

19. An animal monitoring system (500) according to claim 18, wherein said data comprise a photoplethysmogram (601, 602, 603, 604, 605, 606) and said machine learning technology (610) is trained to determine a haemoglobin value (620) for blood of an animal based on said photoplethysmogram (601, 602, 603, 604, 605, 606).
